(19) Europäisches Patentamt
European Patent Office
Office européen des brevets

(11) Publication number: 0 203 559 A1

(12) # EUROPEAN PATENT APPLICATION

(21) Application number: 86107132.2

(22) Date of filing: 26.05.86

(51) Int. Cl.[4]: C 07 D 519/00, C 07 D 499/68, A 61 K 31/43 // (C07D519/00, 499:00, 471:00)

(30) Priority: 27.05.85 IT 2090385

(43) Date of publication of application: 03.12.86 Bulletin 86/49

(84) Designated Contracting States: AT BE CH DE FR GB IT LI LU NL SE

(71) Applicant: AUSONIA FARMACEUTICI S.r.l., Via Laurentina Km. 24730, I-00040 Pomezia (Rome) (IT)

(72) Inventor: Ravagnan, Giampiero, Via Degli Estensi, 291, Roma (IT)

(74) Representative: Bianchetti, Giuseppe, Studio Consulenza Brevettuale Via Rossini, 8, I-20122 Milan (IT)

(54) **Penicillin derivative containing naphthyridine.**

(57) 6 D-(−)-α- [(1-Ethyl- 1,4-dihydro- 7-methyl- 4-oxo-1,8-naphthyridine-3- carboxyamido) -α-(p-hydroxyphenyl)-acetamido] penicillanic acid, prepared by reacting amoxicillin and a reactive derivative of nalidixic acid, has strong antibacterial activity even on resistant strains and good stability towards tryptic enzymes and β-lactamases; and it is suited for oral, parenteral or topical administration, optionally in combination with other penicillins or cephalosporins.

EP 0 203 559 A1

# QUINOLINE-LACTAMINE DERIVATIVE HAVING ANTIBACTERIAL AND ß-LACTAMASES INHIBITORY ACTIVITY

The present invention relates to a novel penicillanic derivative, having specific antibacterial activity against Gram positive and Gram negative bacterial strains producing ß-lactamases as well as against strains resistant to the commonly used ß-lactam antibiotics.

The compound object of the present invention is D-(-)-α-/(1-ethyl-1,4-dihydro-7-methyl-4-oxo-1,8-naphthyridine-3-carboxyamido)-α-(p-hydroxyphenyl)-acetamido/penicillanic acid, of formula I

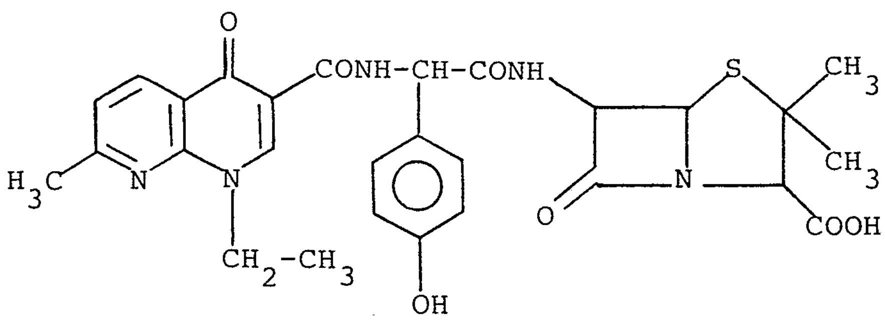

(I)

and pharmaceutically acceptable salts thereof.

Compound I may be prepared by reacting 6-/D(-)-α-amino-p-hydroxyphenyl-acetamido/penicillanic acid, known as Amoxicillin, and a reactive derivative of 1-ethyl-1,4-dihydro-7-methyl-4-oxo-1,8-naphthyridine-3-carboxylic acid (nalidixic acid), such as the acyl chloride or the mixed anhydride.

The reaction is usually carried out in anhydrous aprotic solvents, optionally in the presence of acid acceptor agents, where the acyl chloride is used as the reactive derivative, at a temperature ranging from -10 to

+10°C, preferably from 0 to +5°C.

Compound I proved to have valuable microbiological characteristics, which make it useful in therapy in the treatment of Gram positive and Gram negative bacteria supported infections in humans and warm blood animals.

The minimal inhibitory concentrations (MIC) against some Gram + and Gram - amoxicillin resistant bacteria (amoxicillin MIC > 800 μg/ml) are reported in Table 1 hereinbelow:

**TABLE 1 - Minimal inhibitory concentrations of compound I.**

| Species | N° of strains | MIC (average value) μg/ml |
|---|---|---|
| E. coli | 6 | 25 |
| Proteus mirabilis | 3 | 25 |
| Pseudomas aeruginosa | 5 | 50 |
| Serratia marcescens | 2 | 50 |
| Staph. albus | 1 | 6.25 |
| Staph. aureus CH1 | 1 | 1.56 |
| Staph. aureus coag. pos. | 4 | 3.12 |

These results clearly show a marked antibacterial activity, particularly against penicillinase-producing Staphylococcus strains. Very interesting is also the activity exerted against Pseudomonas aeruginosa strains, which are known to be resistant to Amoxicillin, to ß-lactamic antibiotics and generally to quinoline-like compounds, which usually show no significant activity against Gram-positive bacteria.

Compound I, whilst deriving from two well-known antibacterial agents, is endowed with superior antibacterial properties, which do not derive from those of amoxicillin and of nalidixic acid.

Compound I, therefore, must be considered in any aspect a new derivative of 6-amino-penicillanic acid, also taking into account its remarkable stability.

In order to further evaluate its therapeutic potentiality, compound I was tested for any inhibitory activity against ß-lactamases.

Table 2 hereinbelows shows the obtained results, which evidence that compound I is a strong inhibitory agent of ß-lactamases from both Gram-positive and Gram-negative bacteria, said compound completely inhibiting any enzymatic activity in a dosage of 100 $\gamma$.

TABLE 2

% inhibition by compound I against hydrolysis rates of ß-lactamases

| ß-Lactamase isolated from: | Conc. | % inhibition |
|---|---|---|
| B. cereus | 50 $\gamma$/ml<br>75 $\gamma$/ml<br>100 $\gamma$/ml | 50%<br>90%<br>100% |
| E. coli | 50 $\gamma$/ml<br>75 $\gamma$/ml<br>100 $\gamma$/ml | 50%<br>90%<br>100% |

Therefore, compound I may be suitably used in combination with other ß-lactamic antibiotics, in order to obtain a synergistic effect and/or a protection from inactivation of said antibiotics by ß-lactamases, as it was clearly evidenced by comparing the MICs of a 1:1 by weight combination of compound I and amoxicillin with those of the sole compound I, against Gram-positive and Gram-negative strains resistant to amoxicillin alone (MIC ≥ 800 μg/ml).

Table 3 shows the obtained results, which evidence a synergetic action of compound I with amoxicillin.

**TABLE 3**

**In vitro antibacterial activity of compound I in comparison with a 1:1 compound I/amoxicillin combination. Average values of MICs are expressed as γ/ml.**

| Species | N° of strains | Compound I MIC | Compound I + amoxicillin (1:1) MIC |
|---|---|---|---|
| E. coli | 2 | 75 | 12.5 |
| Enterob. cloacee | 2 | 100 | 12.5 |
| Citrob. freundii | 2 | 100 | 25 |
| Pseudomonas aeruginosa | 5 | 50 | 9.37 |
| Klebsiella pneumoniae | 4 | 87.5 | 5.85 |
| Serratia marcescens | 2 | 50 | 6.25 |
| Salmonella enteriditis | 2 | 100 | 25 |
| Staph. albus | 1 | 6.25 | 1.56 |
| Staph. aureus | 8 | 10.34 | 1.56 |
| Proteus rettgeri | 1 | 50 | 25 |

Another advantageous feature of compound I resides in its ability to inhibit the transfer of bacterial plasmids by conjugation, said ability being shared by other compounds of quinoline structure and being important since it interferes with transmission of bacterial resistance.

The bacterial strains and plasmids listed in Table 4 were used. Phenotype symbols according Novick et al. (1976) were used.

**TABLE 4 - Bacterial strains and plasmids**

| Strains | |
|---|---|
| ZM46 | $Ara^-Lac^-Pro^-Bi^-Nal^+$ |
| UR 10(*) | $Ara^-Lac^-Pro^-Bi^-Rif^+$ |
| CSH50 | $Ara^-Lac^-Pro^-Str^+Bi^-$ |
| CSH28 | $Su3(SuIII^+)Tro^-Ura^-$ $His^-Str^+Bi^-$ |
| **Plasmids** | |
| F'lacpro | LacPro Inc FI |
| RP4 | TcKmAp Inc P |
| R446 | TcSm Inc M |

(*) UR 10 is a rifampicin-resistant mutant of CSH26 strain.

The following antibiotics were used: streptomycin (Sm), tetracycline (Tc), ampicillin (Ap), kanamycin (Km) and rifampicin (Rif). Compound I in 1 and 10 mcg/ml concentrations was used in the transfer mixtures.

- Microorganisms were grown in Nutrient Broth (B.B.L.), NB, or in Nutrient Agar or Mc Conkey Agar (B.B.L.). Appropriate antibiotic concentrations were added to the selection plates. Sm 10, Tc 10, Ap 30, Km 30, Rif 100 and Nal 50.
- Bacterial strains were incubated till $2.5 \times 10^8$ cells/ml of nutrient broth exponential phase, and crossed in a donor/recipient 1:5 ratio in 2 x broth during 18 hours at

37°C. On the other hand, in the interrupted crosses, the bacteria were stirred by means of the Vortex apparatus for 1 minute after 1 h cross.

- About one hundred colonies, which could growth on the selection plates (containing the various antibiotics) were checked for the resistance spectrum by "replica plating".
- The transfer frequence was expressed as the number of resistent recipients in comparison with the number of donors in the mating mixtures, at the plating time. The inhibition rate of plasmid transfer was evaluated in tests in which, in the mating mixtures, compound I was present in 1 or 10 mcg/ml concentrations.

The activity of compound I was tested in different cross systems between E. coli x E. coli.

Table 5 shows the inhibition percentages of the transfer obtained with three different plasmids of incompatibility groups Inc Fi, Inc P and Inc M. The ability of compound I to reduce the transfer of the tested plasmids is evidenced.

**TABLE 5**

**% Inhibition of the transfer of various plasmids, mediated by compound I**

| Cross strains | Compound I concentration | % Inhibition |
|---|---|---|
| CSH28F'lacproxZM46 | 1 mcg/ml | 40.2 |
| CSH28F'lacproxZM466 | 10 mcg/ml | 69.7 |
| CSH28F'lacproxZM46 | | |
| CSH28F'lacproxZM46 | | |
| CSH5O(RP4)xZM46 | 1 mcg/ml | 36.4 |
| CSH5O(RP4)xZM46 | 10 mcg/ml | 66.2 |
| CSH5O(RP4)xZM46 | | |
| CSH5O(RP4)xZM46 | | |
| UR1O(R446)xZM46 | 1 mcg/ml | 19.1 |
| UR1O(R446)xZM46 | 10 mcg/ml | 74.5 |
| UR1O(R446)xZM46 | | |
| UR1O(R446)xZM46 | | |

The above data evidence how compound I, due to its antibacterial and ß-lactamase inhibitory activity, is particularly valuable for the treatment of both Gram-positive and Gram-negative supported infections, in human and veterinary therapies.

Compound I, besides being active against resistant strains and stable to ß-lactamases, is also stable to tryptic enzymes (trypsin, pepsin, etc.) and it is suitable for topical use, due to its peculiar activity against Staphylococci and Pseudomonas, which are among the more common infecting agents in cutaneous diseases. Moreover, compound I may be conveniently combined with ß-lactamase sensitive cephalosporins or penicillins, in order to obtain a synergetic antibacterial action.

The present invention relates also to all the industrial applications of compound I as a therapeutic agent.

Another object of the invention consists therefore in pharmaceutical compositions containing as the active ingredient 10 to 3000 mg of compound I or equivalent amounts of pharmaceutically acceptable salts thereof, for oral, parenteral or topical administration. Generally, the daily dosage will range from 500 mg to 5.0 g, depending also on age, weight and conditions of the patient.

The following examples are given to further illustrate the invention, without limiting the spirit and scope thereof.

EXAMPLE 1

a) 6-D-(-)-α-[(1-Ethyl-1,4-dihydro-7-methyl-4-oxo-1,8-naphthyridine-3-carboxyamido)-α-(p-hydroxyphenyl)-acetamido]penicillanic acid (I)

6 G (0.0594 mole) of anhydrous triethylamine were added to a suspension of nalidixic acid (13.8 g, 0.0594 mole) in 200 ml of anhydrous dimethylformamide. The reaction mixture was cooled to -30°C, under stirring, and 6.45 g (0.0594 mole) of ethyl chlorocarbonate were quickly added thereto. After 30 minutes at -30°C, a solution of trihydrate amoxicillin (24.6 g, 0.0586 mole) and triethylamine (6 g) in 50 ml of dimethylformamide and 25 ml of water was added. The reaction mixture was stirred for 2 hours, while leaving temperature to raise to the room one, then any solid residue was filtered.

The reaction mixture was poured into 1.5 l of ice-water and acidified to pH 2 with 10% HCl, then, after 3 hours at 5°, under stirring, it was filtered washing with

ice-water. After drying at a temperature of less than 40°C, 25 g of compound I were obtained.

**M.p.**: 185-190°C (decomposition).

**Chromatographic analysis (TLC):** silicagel F 254 plate; eluent: a mixture methanol/isopropanol 7:3; single spot with $R_f$ 0.83; developer: UV and iodine vapours; reaction with ninhydrin: negative.

**Elemental analysis**: $C_{28}H_{29}N_5O_7S$ (MW = 579.64)

| | C | H | N |
|---|---|---|---|
| calc. (%): | 58.02 | 5.04 | 12.08 |
| found (%): | 58.21 | 4.98 | 12.17 |

b) 6-D-(-)-α-/(1-Ethyl-1,4-dihydro-7-methyl-4-oxo-1,8-naphthyridine-3-carboxyamido)-α-(p-hydroxyphenyl)-acetamido/penicillanic acid (I) sodium salt

15.70 Ml of a 10% sodium hydroxide solution were added to 25 g of (I) in deionized water. The mixture was stirred to dissolution and filtered. The filtrate was collected in a stainless steel vessel, to obtain a liquid level of about 1 cm, then it was placed in a freeze-dryer and quickly frozen at -45°C. During the last 8 hours of freeze-drying the temperature was raised to +35°C. 25.94 G of compound I sodium salt were obtained.

**M.p.**: 220°C (decomposition).

**Elemental analysis**: $C_{28}H_{28}N_5O_7SNa$ (MW = 601.62)

| | C | H | N |
|---|---|---|---|
| calc. (%): | 55.90 | 4.69 | 11.64 |
| found (%): | 56.07 | 4.77 | 11.35 |

EXAMPLE 2

According to conventional pharmaceutical techniques, the following compositions were prepared, containing as the active ingredient compound I or its sodium salts, together with appropriate diluents or excipient:

a) injectable sterile ampouls, containing 1.081 g of compound I sodium salt (titre: 96%);

b) 3% ointment;

c) 5% powder;

d) gelatin operculated capsules, containing 250-500 mg of compound I (assay: 100%).

CLAIMS for the Contracting States:

BE, CH, DE, FR, GB, IT, LI, LU, NL, SE

1. 6-D-(-)-α-[(1-Ethyl-1,4-dihydro-7-methyl-4-oxo-1,8-naphthyridine-3-carboxyamido)-α-(p-hydroxyphenyl)-acetamido]penicillanic acid of formula I

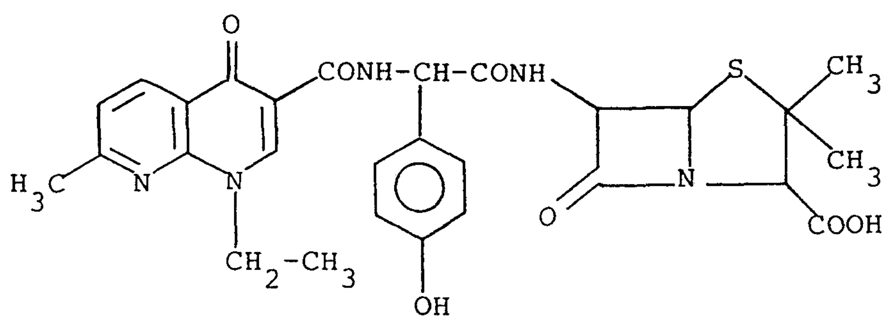

(I)

and pharmaceutically acceptable salts thereof.

2. 6-D-(-)-α-[(1-Ethyl-1,4-dihydro-7-methyl-4-oxo-1,8-naphthyridine-3-carboxyamido)-α-(p-hydroxyphenyl)-acetamido]penicillanic acid sodium salt.

3. A process for preparing compound of claim 1, which process consists in reacting amoxicillin with a nalidixic acid reactive derivative (chloride or anhydride), in aprotic solvents and in the presence of acid-binding agents.

4. Pharmaceutical compositions having antibacterial activity for oral, topical or parenteral administration, containing therapeutically effective amounts of compound I or pharmaceutically acceptable salts thereof.

5. Pharmaceutical compositions as claimed in claim 4, in form of ampoules, capsules, tablets, syrups, creams, ointments, powders.

**CLAIM for AT:**

A process for the preparation of 6-D-(-)-α-/(1-ethyl-1,4-dihydro-7-methyl-4-oxo-1,8-naphthyridine-3-carboxyamido)-α-(p-hydroxyphenyl)-acetamido/penicillanic acid of formula I

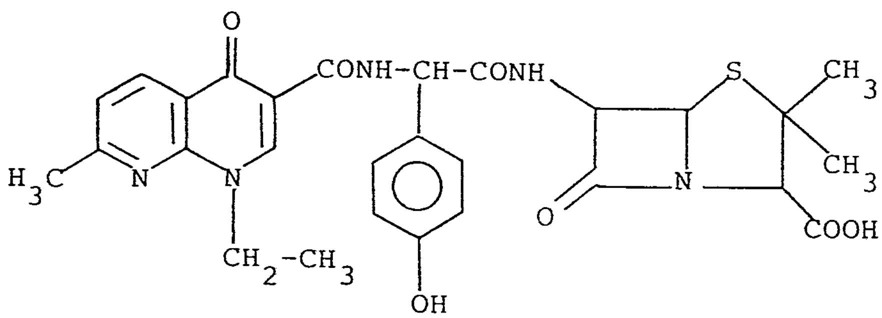

(I)

characterized in that amoxicillin is reacted with a nalidixic acid reactive derivative (chloride or anhydride), in aprotic solvents and in the presence of acid-binding agents.

European Patent Office

# EUROPEAN SEARCH REPORT

0 203 559

Application number

EP 86 10 7132

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | CHEMICAL ABSTRACTS, vol. 97, no. 7, 16th August 1982, page 626, abstract no. 55572w, Columbus, Ohio, US; & JP - A - 82 46 988 (FUJIMOTO PHARMACEUTICAL CO. LTD.) 17-03-1982<br>* Abstract *<br>--- | 1-3 | C 07 D 519/00<br>C 07 D 499/68<br>A 61 K 31/43 //<br>(C 07 D 519/00<br>C 07 D 499:00<br>C 07 D 471:00 ) |
| Y | FR-A-2 210 385 (SUMITOMO)<br>* Pages 19,20; example 7; claims *<br>--- | 1-5 | |
| Y | FR-A-2 191 556 (R. ARIES)<br>* Claims *<br>--- | 1-3 | |
| Y | FR-A-2 183 895 (YAMANOUCHI)<br>* Pages 13,14; example 1; claims *<br>--- | 1,4 | **TECHNICAL FIELDS SEARCHED (Int. Cl.4)** |
| Y | FR-A-2 224 132 (SUMITOMO)<br>* Pages 17,18, example 19; claims *<br>--- | 1-5 | C 07 D 519/00<br>C 07 D 499/00<br>A 61 K 31/00 |
| Y | CHEMICAL ABSTRACTS, vol. 96, no. 25, 21st June 1982, page 718, abstract no. 217590j, Columbus, Ohio, US; JP - A - 82 32 290 (FUJIMOTO PHARMACEUTICAL CO. LTD.) 20-02-1982<br>* Abstract *<br>--- -/- | 1-3 | |

The present search report has been drawn up for all claims

| Place of search | Date of completion of the search | Examiner |
|---|---|---|
| THE HAGUE | 01-09-1986 | CHOULY J. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

**0 203 559**

Application number

EP 86 10 7132

Page 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | CHEMICAL ABSTRACTS, vol. 97, no. 9, 30th August 1982, page 616, abstract no. 72183n, Columbus, Ohio, US; JP - A - 82 46 990 (FUJIMOTO PHARMACEUTICAL CO. LTD.) 17-03-1982<br>* Abstract *<br>----- | 1,3 | |
| | | | TECHNICAL FIELDS SEARCHED (Int. Cl.4) |

The present search report has been drawn up for all claims

| Place of search | Date of completion of the search | Examiner |
|---|---|---|
| THE HAGUE | 01-09-1986 | CHOULY J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82